# EUROPEAN PATENT SPECIFICATION

(11) **EP 1 737 467 B1**
(45) Date of publication and mention of the grant of the patent: **13.07.2011**
(21) Application number: 05732550.8
(22) Date of filing: 29.03.2005
(51) Int. Cl.: A61K 31/4174, A61K 31/137, A61K 31/4402, A61K 31/573, A61K 45/06, A61P 17/00, A61P 17/06, A61P 17/08

(54) **COMPOSITION FOR THE TREATMENT OF SKIN CONDITIONS**
ZUSAMMENSETZUNG ZUR BEHANDLUNG VON HAUTERKRANKUNGEN
COMPOSITION POUR LE TRAITEMENT D'AFFECTIONS DE LA PEAU

(30) Priority: 30.03.2004 US 812809
(43) Date of publication of application: 03.01.2007
(73) Proprietor: Hutterer, Jeffrey H., Forest Hills, NY 11375 (US)
(72) Inventor: Hutterer, Jeffrey H., Forest Hills, NY 11375 (US)
(74) Representative: McCarthy, Denis Alexis
(86) International application number: PCT/US2005/010269
(87) International publication number: WO 2005/097139

(56) References cited:
- GB-A- 1 087 842
- US-A1- 2002 061 281
- US-A1- 2002 106 342
- US-A1- 2004 204 399
- US-A1- 2005 053 635
- US-B2- 6 572 849

## Description

### Field of the Invention

The field of this invention is compositions for the treatment of dermatological conditions and, more particularly, such compositions for the treatment of non-viral and non-bacterial based dermatological conditions such as eczema, seborrheic dermatitis, psoriasis and allergic skin reactions including hives, skin irritations, dermatological conditions caused by poison ivy, and dermatological conditions caused by poison oak.

### Background of the Invention and Discussion of the Prior Art

Certain individuals are prone to dermatological conditions in which a skin outbreak occurs. These outbreaks, which are not known to be caused primarily by a virus or bacteria, include eczema, seborrheic dermatitis, psoriasis and allergic skin reactions such as hives, skin irritations, dermatological conditions caused by poison ivy and dermatological conditions caused by poison oak. They can be embarrassing when they occur and quelling them is obviously a compelling need. For certain individuals, the need to alleviate such skin conditions is not only social but also business-related. For example, if an actor had such a skin eruption while on the set, he or she would need to alleviate the condition immediately.

Certain compositions are well known for the treatment of skin conditions not known to be caused primarily by a virus such as eczema, seborrheic dermatitis, psoriasis and allergic skin reactions such as hives, skin irritations, dermatoloical conditions aused by poison ivy and dermatological conditions caused by poison oak. In particular, topical corticosteroids are well known for the treatment of these conditions. These compositions come in the form of creams, ointments and lotions.

Topical corticosteroids are rated according to potency from Class I to Class VII. Class I corticosteroids are considered superpotent formulations and they are about 1,000 times stronger than 1% hydrocortisone, which is a Class VII formulation.

U.S. Patent No. 4,256,763 to McHugh reports that in the use of phenolphthalein to fight inflammatory viral infections, antihistamines, decongestants, analgesics and antipyretics have been successfully combined with phenolphthalein to relieve other cold and flu symptoms often associated with the inflammatory viral inflections.

In contrast to the anti-inflammatory compound phenolphthalein, it is generally recognized that topical corticosteroids, e.g. hydrocortisone 1% and others, are not prescribed for viral based or bacterial based skin inflammations, although they are prescribed for certain skin conditions not known to be primarily caused by virus or bacteria. The reason is that corticosteroids suppress the immune system. Hence, there is a difference between what is prescribed for viral-based and bacterial-based inflammatory skin conditions and those not known to be primarily caused by a virus or bacteria, such as eczema, seborrheic dermatitis, psoriasis and allergic skin reactions such as hives, skin irritations, dermatological conditions caused by poison ivy, dermatological conditions caused by poison oak, and the like. With respect to the latter skin conditions, for which corticosteroids are prescribed, there is a compelling need for ways to enhance the effectiveness and absorption of such steroids.

U.S. Patent No. 5,164,406 to Helman teaches adding imidazole or imidazole derivatives (one such derivative is listed as naphazoline HCL) to compositions containing active pharmacological agents in order to increase the transdermal penetration of the compound. Helman lists a wide array of such pharmacological agents that in principal should experience enhanced transdermal penetration and steroids including hydrocortisone are listed as one category that should benefit. Helman's ten laboratory examples, however, do not evidence tests actually performed on hydrocortisone or on other topical corticosteroids.

Applicant has experimented significantly over many years with methods of enhancing the effectiveness and absorbability of topical corticosteroids in the treatment of eczema, seborrheic dermatitis, psoriasis and allergic skin reactions and in general with treatments for these conditions. Applicant found that the application of naphazoline HCL alone or phenylephrine HCL alone provides a mildly effective alleviation of the symptoms of these conditions. Applicant also found that the application of the combination of naphazoline HCL and pheniramine maleate with no steroid was more effective in alleviating the symptoms of these conditions than naphazoline HCL alone or phenylephrine HCL alone. The level of effectiveness was comparable to the use of hydrocortisone 1% alone. Similarly, Applicant also found that the application of the combination of phenylephrine HCL and pheniramine maleate with no steroid was more effective in alleviating the symptoms of these conditions than phenylephrine HCL alone and than naphazoline HCL alone. The level of effectiveness was comparable to the use of hydrocortisone 1% alone.

Applicant also experimented by using these substances to enhance the effectiveness of the topical corticosteroids. Applicant applied solutions containing naphazoline HCL as the only active ingredient prior to the application of the corticosteroid to the affected skin area. The results of these experiments showed somewhat beneficial results were achieved regarding effectiveness and absorbability from the application of naphazoline HCL prior to the application of the corticosteroid to the affected area. The effectiveness was slightly greater than that of a topical corticosteroid alone. Numerous other compounds were also tried. In sum, dramatic increases in effectiveness and/or absorbability of a topical corticosteroid were not achieved by Applicant's experiments except by the use of the compositions of the present invention, as will be described.

Certain antihistamines claim to be indicated for the temporary relief of itching and pain associated with insect bites, minor skin irritations, rashes due to poison ivy, poison oak or poison sumac. They are prescribed to treat itching caused by dryness on the skin. For example, Benadryl® Extra Strength Itch Stopping Cream claims to work for skin itching. Benadryl® contains Diphenhydramine Hydrochloride 2% and zinc acetate 0.1%. However, diphenhydramine hydrochloride is an antihistamine with drying and sedative side effects. It also has significant side effects from overdosage in pediatric patients. Therefore, Benadryl®, even in the cream form, is not indicated for children and would not be a recommended choice for preparing the skin to enhance the effectiveness of topical corticosteroids. Furthermore, Applicant has experimented with applying Benadryl® to the affected area for the above-mentioned skin conditions (eczema, etc.) prior to the application of a topical corticosteroid and has not found significant enhancement of effectiveness.

Antihistamine and decongestant combinations are well-known for the treatment of nasal congestion (stuffy nose), sneezing, and runny nose caused by colds and hay fever. Antihistamines work by preventing the effects of a substance called histamine, which is produced by the body. Histamine can cause itching, sneezing, runny nose, and watery eyes. Antihistamines contained in these combinations are acrivastine, azatadine, brompheniramine, carbinoxamine, chlorpheniramine, clemastine, dexbrompheniramine, diphenhydramine, loratadine, pheniramine, phenyltoloxamine, promethazine, pyrilamine, and triprolidine. The decongestants, such as phenylephrine, and pseudoephedrine, produce a narrowing of blood vessels. This leads to clearing of nasal congestion, although it may also cause an increase in blood pressure in patients who have high blood pressure. To Applicant's knowledge, it is not known to use antihistamine and decongestant combinations to successfully and dramatically increase the effectiveness and absorbability of topical corticosteroids.

What is needed is a way to dramatically treat eczema, seborrheic dermatitis, psoriasis, hives, skin irritations, dermatological conditions caused by poison ivy, dermatological conditions caused by poison oak, and other dermatological conditions not known to be caused primarily by a virus or bacteria. Since topical corticosteroids are the medications of choice for the treatment of these conditions, there is also a compelling need for ways to enhance the effectiveness and absorption of topical corticosteroids prescribed for eczema, seborrheic dermatitis, psoriasis, hives, skin irritations, dermatological conditions caused by poison ivy, dermatological conditions caused by poison oak, and other dermatological conditions not known to be caused primarily by a virus or bacteria.

### SUMMARY OF THE PRESENT INVENTION

Over the course of at least four decades, Applicant, has as an adult male patient, due to his sensitive skin, endured numerous dermatological conditions such as eczema, seborrheic dermatitis and allergic skin reactions such as hives, skin irritations, reactions to plants, etc. As a result he has experimented with wide range of known topical corticosteroid creams that are available by prescription or over-the-counter, as well as numerous topical corticosteroid ointments and lotions. The experiments were conducted in order to try to treat these conditions as well as to try to enhance the performance of hydrocortisone and other topical corticosteroids in the treatment of these conditions.

The experiments have in general already been described. With respect to the use of compounds to try to enhance the effectiveness of topical corticosteroids, the experiments involved first applying to the affected area particular compounds and then shortly thereafter applying to the same affected area a topical corticosteroid. For example, compounds that were tried include eye drop solutions, nasal decongestant sprays and other kinds of compounds available over the counter or by prescription. Antihistamine and decongestant combinations were tried as a solution to be applied to the affected area prior to the application of the steroid. In addition, antihistamines alone (preceding the corticosteroid) and decongestants alone (preceding the corticosteroid) were tried. As stated above, antihistamines alone (without the corticosteroid) and decongestants alone (without the corticosteroid) were also tried.

An increase in effectiveness is defined as greater alleviation of the dermatological symptoms of the condition or speedier alleviation of the dermatological symptoms of the condition or both.

Among the many compounds experimented with, the patient tried applying solutions containing naphazoline HCL alone as the only active ingredient and solutions containing phenylephrine HCL alone as the only active ingredient, in each case either prior to the application of the topical corticosteroid cream or alone. When naphazoline HCL was applied as the only active ingredient of a solution and when phenylephrine HCL was applied as the only active ingredient of a solution the results were only mild enhancement of effectiveness of the topical corticosteroid. When Benadryl containing diphenhydramine HCL, an antihistamine, was tried as the only active ingredient (except for a small amount of zinc acetate) there was no appreciable increase in effectiveness.

Applicant also tried oxymetazoline HCL decongestant alone in the form of a nasal decongestant Vicks® Sinex® and the results were not beneficial.

There was an exception to these disappointing results. Applicant found that when he applied a combination of pheniramine maleate and naphazoline HCL or a combination of pheniramine maleate and phenylephrine HCL (in each case together with other inactive ingredients and/or preservatives) and he then applied a topical corticosteroid cream, the results were startling. Increased effectiveness both in terms of speed and in terms of greater alleviation of symptoms were observed as compared to the use of a topical corticosteroid alone. Furthermore, the relief obtained was also longer lasting by approximately one hour. That is whereas a topical corticosteroid relieve dermatological symptoms of the described conditions for one to three hours, the present invention provides such relief for approximately two to five hours.

As a result of the longer lasting relief provided by the present invention, money is also saved since the user need apply the medication fewer times a day.

Accordingly, one aspect of the present invention is a composition for treating dermatological conditions not known to be caused by a virus or bacteria selected from eczema, seborrheic dermatitis, psoriasis, allergic skin reactions, hives, skin irritations, dermatological conditions caused by poison ivy, dermatological conditions caused by poison oak, comprising a cream whose active ingredients comprise (1) naphazoline HCL or phenylephrine HCL, (2) pheniramine maleate, and (3) a topical corticosteroid.

In one preferred embodiment, the topical corticosteroid is hydrocortisone. In another preferred embodiment, the topical corticosteroid is selected from the group consisting of halobetasol propionate, desoximetasone, betamethasone diproprionate, beta methasone valerate and triamcinolone acetonide.Applicant has discovered what appears to be a synergistic effect that is not fully understood that arises from applying approximately one to five drops of a solution containing the combination of pheniramine maleate with either naphazoline HCL or phenylephrine HCL, such as in the form of an eye drops solution containing these two active ingredients, on to the collection of affected areas of the skin condition just prior to applying a cream containing hydrocortisone or other topical corticosteroid on to the same affected area. The result is a dramatic alleviation of the symptoms of the condition and a dramatically increased effectiveness in comparison of the topical corticosteroid cream alone both in terms of degree of symptom alleviation and in terms of the speed of said symptom alleviation. Generally, the dermatological condition is remarkably alleviated in two to four minutes. In terms of speed, this is less than half the time, on average that it would take using a cream of hydrocortisone or other topical corticosteroid on the affected area alone.

As a result of some unknown synergistic effect between the pheniramine maleate, the naphazoline HCL or the phenylephrine HCL, and the hydrocortisone, the composition of the present invention produces the remarkable and startling result that the symptoms of thedermatological condition disappears or virtually disappears in approximately two to four minutes. Applicant also detected no side effects and believes the composition of the present invention to be completely safe. The result Applicant found surpasses the effectiveness of any known prescribed or over the counter topical corticosteroid cream, ointment or lotion in the treatment of eczema, seborrheic dermatitis, psoriasis, hives, skin irritations, dermatological conditions caused by poison ivy, dermatological conditions caused by poison oak, and other dermatological conditions not known to be primarily caused by a virus or bacteria.

Although the above effect is not fully understood, Applicant notes the following in hindsight. In times of stress the adrenal gland of the body secretes two classes of hormones. The adrenal medulla releases epinephrine and nor-epinephrine (the prototypes of all decongestants). Their primary purpose is to prepare the body for "fight or flight". The adrenal cortex emits corticosteroids, from which the synthetic corticosteroids, including hydrocortisone, have been derived. They function to preserve metabolic homeostasis during emergencies. Since the same adrenal gland secretes these hormones, which have naturally evolved to work in concert, and which both serve to quell allergic and inflammatory reactions, it is logical in hindsight that combinations of decongestants and corticosteroids would function effectively in topical preparations.

### IMPORTANT OBJECTS AND ADVANTAGES

The following important objects and advantages of the present invention are:
(1) to provide a method of dramatically alleviating the symptoms of non-viral and non-bacterial based dermatological conditions such as eczema, seborrheic dermatitis, psoriasis and allergic skin reactions such as hives, skin irritations, dermatological conditions caused by poison ivy, dermatological conditions caused by poison oak;
(2) to provide a composition for speedily alleviating the symptoms of non-viral and non-bacterial based dermatological conditions such as eczema, seborrheic dermatitis, psoriasis and allergic skin reactions such as hives, skin irritations, dermatological conditions caused by poison ivy, dermatological conditions caused by poison oak;
(3) to provide a composition for dramatically increasing the effectiveness and absorption of topical corticosteroids for use in the treatment of non-viral and non-bacterial based dermatological conditions;
(4) to provide a composition for treating the above dermatological conditions that increases the absorption of the topical corticosteroid;
(5) to provide a composition for treating the above dermatological conditions that alleviates the symptoms of the skin condition much faster than known compositions;
(6) to provide a composition for treating the above dermatological conditions that alleviates the symptoms of the skin condition in approximately 2 to 4 minutes;
(7) to provide a composition for treating the above dermatological conditions that can be applied using non-prescription medications;
(8) to provide a composition for treating the above dermatological conditions that can be applied using easily accessible medications such as eye drops;
(9) to provide a composition for treating the above dermatological conditions that takes only a few seconds to employ;
(10) to provide a composition for enhancing the performance of topical corticosteroids in the treatment of the above dermatological conditions without the need to employ increased concentrations of topical corticosteroids to achieve a desired level of effectiveness and/or absorption;
(11) to provide a composition for treating such dermatological conditions by enhancing the effectiveness and absorbability of topical corticosteroids without the need to create any new formulations or compounds by relying on existing formulations and compounds;
(12) to provide a composition for dramatically increasing the effectiveness and absorption of topical corticosteroids for use in the treatment of non-viral and non-bacterial based dermatological conditions without serious side effects;
(13) to provide a composition for dramatically increasing the effectiveness and absorption of topical corticosteroids for use in the treatment of non-viral and non-bacterial based dermatological conditions without generating any side effects not generated from the use of the topical corticosteroid alone; and
(14) to provide a composition for treatment of the symptoms of non-viral and non-bacterial based dermatological conditions such as eczema, seborrheic dermatitis, psoriasis and allergic skin reactions such as hives, skin irritations, dermatological conditions caused by poison ivy, dermatological conditions caused by poison oak that provides longer lasting relief from the symptoms of these dermatological conditions than does the application of topical corticosteroids alone;
(15) to provide a composition for treatment of the symptoms of non-viral and non-bacterial based dermatological conditions such as eczema, seborrheic dermatitis, psoriasis and allergic skin reactions such as hives, skin irritations, dermatological conditions caused by poison ivy, dermatological conditions caused by poison oak that provides longer lasting relief from the symptoms of these dermatological conditions than does the application of topical corticosteroids;
(16) to provide a composition for treatment of the symptoms of non-viral and non-bacterial based dermatological conditions such as eczema, seborrheic dermatitis, psoriasis and allergic skin reactions such as hives, skin irritations, dermatological conditions caused by poison ivy, dermatological conditions caused by poison oak that requires fewer applications per day and hence provides cost savings as a result of the longer lasting relief from the symptoms of these dermatological conditions than does the application of topical corticosteroids; and
(17) to provide a composition for treatment of the symptoms of non-viral and non-bacterial based dermatological conditions such as eczema, seborrheic dermatitis, psoriasis and allergic skin reactions such as hives, skin irritations, dermatological conditions caused by poison ivy, dermatological conditions caused by poison oak that works with topical corticosteroids of all the potency classes (Class I through Class VII).

### DETAILED DESCRIPTION OF THE PREFERRED EMBODIMENT

The method of using the composition of the present invention will now be illustrated in further detail, including through the provision of examples.

Topical corticosteroids are rated according to potency from Class I to Class VII. Class I corticosteroids are considered superpotent formulations and they are about 1,000 times stronger than 1% hydrocortisone, which is a Class VII formulation. The following topical corticosteroids form a partial list of those that have been tested with the method of the present invention.
Class I Halobetasol propionate 0.05% Ultravate® Cream
Class II Desoximetasone 0.24% Topicort® Cream
   Betamethasone diproprionate 0.05% Diprolene® Cream AF
Class V Beta methasone valerate Valisone® Cream
   Hydrocortisone valerate 0.2% Westcort® Cream
   Triamcinolone acetonide 0.25% Aristicort® Cream
Class VII Hydrocortisone 2.5% Hytone® Ointment 2.5%
   Hydrocortisone 1.0% Hytone® Cream 1%
   Synacort® Cream 1 %
   Cort-Aid® Cream 1%
   Cortizone® Cream 1%
   Maximum Strength Hydrocortisone Cream 1% by Duane Reade

The method of using the composition of the present invention involves two steps. The first step involves applying a liquid mixture of an antihistamine and a decongestant to the affected area of the skin. The mixture is applied through a dropper or other suitable applicator on to the skin. The quantity applied would depend upon the quantity of steroid cream expected to be applied. The preferred ratio is approximately one drop of solution for every approximately 0.1 grams of cream that is expected to be applied to the affected area in step two of the method of the present invention. The weight of approximately 0.1 grams roughly corresponds to a smear from an average-sized dispenser of topical corticosteroid cream that is roughly one sixteenth of an inch long to approximately one eighth of an inch long.

Deviations with respect to each component (solution and cream) from the preferred ratio by a factor of two or three would still be expected to achieve a significant portion of the results achieved by the method of the present invention.

A "drop" of solution is approximately 0.06 milliliters. This measurement of a drop is based upon the quantity of solution that exists in an average drop of ophthalmic solution such as Opcon-A® Typically, the amount of solution applied to the entire collection of affected areas of skin will be one to five drops (between approximately 0.06 milliliters and 0.30 milliliters) and preferably two to three drops. This is simply an estimate of the number of patches of affected areas of skin sufferers of these conditions typically have, so obviously this amount can vary. A "patch" of an affected area of skin is taken to refer approximately to an area of skin between approximately one half of a square inch to approximately one square inch.

If in keeping the above method a total two to three drops were to be applied to the entire collection of affected areas of skin on the person, i.e., the plurality of patches of affected area, then that would amount to applying between approximately 0.12 milliliters and approximately 0.18 milliliters of solution, and this would then correlate with a preferred usage of topical corticosteroid cream corresponding to between approximately 0.2 and 0.3 grams.

If the applicator is in the form of a spray, such as Dristan® Nasal Spray ("fast acting formula"), the spray bottle should be turned upside down and a few drops allowed to drop out, thus converting the applicator to a dropper. Alternatively, if the spray applicator is kept in the form of a spray, the quantity applied should be the amount of spray from a quick blast of approximately half a second. The aforementioned "approximately half a second" quantity is a very rough estimate of the appropriate time needed.

The method of using the composition of the present invention was not used for inflammations that are viral infections like Herpes Simplex infections since topical corticosteroids are not the preferred agent for the treatment of such inflammations since they suppress the immune system.

It is contemplated by the method of using the composition of the present invention that the solution utilized in the first step of said method can be obtained from a known pharmaceutical product either in the form of an ophthalmic solution, a nasal decongestant solution or spray or any other suitable mixture. It is also contemplated by the present invention that the solution utilized in the first step of the method of the present invention can be created specifically for the purposes of the present invention. In that case, it is easily discernable by one skilled in the art to add the appropriate amounts of preservatives, pH adjusters and other inactive ingredients to the solution.

After the first step, or as part of the first step, either wait a few seconds for the solution to penetrate the skin or else smear it with one's finger or other appropriate object to make sure it penetrates.

The second step involves applying the topical corticosteroid cream (or lotion or ointment) in the normal manner prescribed for, which is to simply smear an appropriate quantity on to the skin in the same affected area as the solution was applied to.

In the below examples, the phrase "alleviation of symptoms" means alleviation of both the visual dermatological symptoms as well as the alleviation of the other symptoms such as the pain and itching. The term "patch" in the below examples refers to an area of skin of approximately one half of a square inch to approximately one square inch.

It is important to note with respect to the method of using the composition of the present invention (and associated composition) that it contemplates implementing the method either by first applying the appropriate drop of solution to a single particular patch of affected area of skin and then applying an appropriate amount of the topical corticosteroid cream to that particular single patch of affected area of skin or else by applying all the appropriate drops of solution to all the patches of affected area of skin and then smearing the appropriate amount of cream on all the patches. This is because the usefulness of the applied solution is not compromised by waiting a few seconds more or less before applying the cream. The method further contemplates utilizing any in-between combination of the above two described procedures.

### EXAMPLE I

In order to treat a collection of patches of eczema, one drop of an eye drops solution called Opcon-A® was applied to each patch of affected area of the skin and allowed to penetrate the skin. After a few seconds of the application of each drop of solution, hydrocortisone 1% cream under a well known brand name such as Hytone® Cream, a Class VII topical corticosteroid, was applied to the same affected area. The quantity applied to each patch is a smear approximately one sixteenth of an inch to one eighth of an inch long for each patch and weighs approximately 0.1 grams. The aforementioned Opcon-A eye drops solution is a mixture of the below ingredients, formulated as follows:

| Ingredient | Percent |
|---|---|
| 1. Naphazoline HCL | approximately 0.03% |
| 2. Pheniramine maleate | approximately 0.32% |
| 3. Benzalkonium chloride | approximately 0.1% |
| 4. Boric Acid | |
| 5. Edetate disodium | approximately 0.1% |
| 6. Hydroxypropylmethylcellulose | |
| 7. Purified water | |
| 8. Sodium borate | |
| 9. Sodium chloride | |

The result was that the patch of eczema on the patient's skin was dramatically alleviated in approximately three minutes and this relief lasted approximately three hours.

### EXAMPLE II

In order to treat a collection of patches of seborrheic dermatitis, approximately one drop of an eye drops solution called Opcon-A® was applied to each patch of the affected area of the skin and allowed to penetrate the skin. After a few seconds, betamethasone valerate under the brand name Valisone® Cream, a Class V topical corticosteroid, was applied to the same affected area. The quantity applied for each small patch is a smear approximately one eighth to one sixteenth inch long. The aforementioned Opcon-A eye drops solution is a mixture of the below ingredients, formulated as follows The solution is made of the liquid mixture of the below ingredients, formulated as follows:

| Ingredient | Percent |
|---|---|
| 1. Naphazoline HCL | approximately 0.03% |
| 2. Pheniramine maleate | approximately 0.32% |
| 3. Benzalkonium chloride | approximately 0.1% |
| 4. Boric Acid | |
| 5. Edetate disodium | approximately 0.1 % |
| 6. Hydroxypropylmethylcellulose | |
| 7. Purified water | |
| 8. Sodium borate | |
| 9. Sodium chloride | |

The result was that the patch of seborrheic dermatitis on the patient's skin was dramatically alleviated in approximately two to three minutes and the relief lasted approximately four hours.

### EXAMPLE III

In order to treat a group of patches of seborrheic dermatitis, for approximately half a second a spray of a nasal decongestant Dristan® Nasal Spray ("fast acting formula") was sprayed on each patch of the affected area of the skin and allowed to penetrate the skin. After a few seconds, hydrocortisone valerate under the brand name Westcort® Cream, a Class V topical corticosteroid, was applied to the same affected area. The quantity applied for each patch of affected area of skin is a smear approximately one sixteenth of an inch to one eighth of an inch long. The aforementioned Dristan Nasal Spray solution is a mixture of the below active ingredients, formulated as follows:

| Ingredient | Percent |
|---|---|
| 1. Phenylephrine HCL | approximately 0.50% |
| 2. Pheniramine maleate | approximately 0.20% |

The result was that the manifestations of seborrheic dermatitis on the patient's skin was dramatically alleviated in approximately two to four minutes and the relief lasted for approximately three to four hours.

### EXAMPLE IV

In order to treat a group of patches of eczema, one drop of an eye drops solution called Opcon-A® was applied to each patch of the affected area of the skin and allowed to penetrate the skin. After a few seconds, hydrocortisone 2.5% under the brand name Hytone® Ointment, a Class VII topical corticosteroid, was applied to the same affected area. The quantity applied per patch of affected area is a smear approximately one sixteenth to one eighth of an inch long. The weight of the cream applied is approximately 0.1 grams. The aforementioned Opcon-A eye drops solution is a mixture of the below ingredients, formulated as follows:

| Ingredient | Percent |
|---|---|
| 1. Naphazoline HCL | approximately 0.03% |
| 2. Pheniramine maleate | approximately 0.32% |
| 3. Benzalkonium chloride | approximately 0.1 % |
| 4. Boric Acid | |
| 5. Edetate disodium | approximately 0.1 % |
| 6. Hydroxypropylmethylcellulose | |
| 7. Purified water | |
| 8. Sodium borate | |
| 9. Sodium chloride | |

The result was that the symptoms of eczema on the patient's skin almost completely cleared up in approximately three minutes and the relief lasted for approximately five hours.

### EXAMPLE V

In order to treat an allergic skin reaction occurring in an approximately three square inch area of skin, for example hives, three drops of an eye drops solution called Opcon-A® was applied to the entire affected area of the skin and allowed to penetrate the skin. After a few seconds, triamcinolone acetonide 0.25% under the brand name Aristicort® Cream, a Class V topical corticosteroid, was applied to the same affected area. The quantity applied is a smear approximately one eighth to one sixteenth inch long per approximately square inch of affected area of skin. The aforementioned Opcon-A eye drops solution is a mixture of the below ingredients, formulated as follows:

| Ingredient | Percent |
|---|---|
| 1. Naphazoline HCL | approximately 0.03% |
| 2. Pheniramine maleate | approximately 0.32% |
| 3. Benzalkonium chloride | approximately 0.1% |
| 4. Boric Acid | |
| 5. Edetate disodium | approximately 0.1 % |
| 6. Hydroxypropylmethylcellulose | |
| 7. Purified water | |
| 8. Sodium borate | |
| 9. Sodium chloride | |

The result was that the hives on the patient's skin almost completely cleared up in approximately four minutes and the relief lasted for approximately three hours.

### EXAMPLE VI

In order to treat a group pf patches of seborrheic dermatitis, a bottle of the nasal decongestant Dristan® Nasal Spray ("fast acting formula") was held upside down and approximately one drop was allowed to drop on to each patch of the affected area of the skin and allowed to penetrate the skin. After a few seconds, desoximetasone 0.25% under the brand name Topicort® Cream, a Class V topical corticosteroid, was applied to the same affected area. The quantity applied for each patch is a smear approximately one sixteenth of an inch long. The aforementioned Dristan Nasal Spray solution is a mixture of the below active ingredients, formulated as follows:

| Ingredient | Percent |
|---|---|
| 1. Phenylephrine HCL | approximately 0.50% |
| 2. Pheniramine maleate | approximately 0.20% |

The result was that the patches of seborrheic dermatitis on the patient's skin was dramatically alleviated in approximately two minutes and the relief lasted for approximately two to three hours.

### EXAMPLE VII

In order to treat a collection of patches of seborrheic dermatitis, a drop of a nasal decongestant Dristan® Nasal Spray was dropped on to each patch of the affected area of the skin and allowed to penetrate the skin by holding the container upside down. After a few seconds, hydrocortisone 1% under the brand name Hytone® Cream 1%, a Class VII topical corticosteroid, was applied to the same affected area. The quantity applied for each patch is a smear approximately one sixteenth of an inch long. The aforementioned Dristan Nasal Spray solution contains the below active ingredients:

| Ingredient | Percent |
|---|---|
| 1. Phenylephrine HCL | approximately 0.50% |
| 2. Pheniramine maleate | approximately 0.20% |

The result was that the symptoms of the seborrheic dermatitis on the patient's skin was dramatically alleviated in approximately two to four minutes. This relief lasted over three hours.

### EXAMPLE VIII

In order to treat a group of patches of eczema, a drop of an eye drops solution called Opcon-A® was applied to each patch of the affected area of the skin and allowed to penetrate the skin. After a few seconds, Halobetasol propionate 0.05% under a well known brand name such as Class I Ultravate® Cream, a Class I topical corticosteroid, was applied to the same affected area. The quantity applied per patch is a smear approximately one eighth to approximately sixteenth of an inch long and weighs approximately 0.1 grams. The aforementioned Opcon-A eye drops solution is a mixture of the below ingredients, formulated as follows:

| Ingredient | Percent |
|---|---|
| 1. Naphazoline HCL | approximately 0.03% |
| 2. Pheniramine maleate | approximately 0.32% |
| 3. Benzalkonium chloride | approximately 0.1% |
| 4. Boric Acid | |
| 5. Edetate disodium | approximately 0.1 % |
| 6. Hydroxypropylmethylcellulose | |
| 7. Purified water | |
| 8. Sodium borate | |
| 9. Sodium chloride | |

The result was that the patches of eczema on the patient's skin were dramatically alleviated in approximately three minutes and this relief lasted approximately three hours.

### EXAMPLE IX

In order to treat a group of patches of seborrheic dermatitis, hydrocortisone 1 % under the brand name Hytone® Cream 1%, a Class VII topical corticosteroid, was applied to the same affected area. The quantity applied for each patch is a smear approximately one sixteenth of an inch long. The result was that the seborrheic dermatitis was improved although not dramatically in approximately seven minutes. The relief lasted for two hours.

### SUMMARY OF EXAMPLES X THROUGH XVI

Each one of examples I through VI and example VIII listed above illustrating the method of using the composition of the present invention can be compared with examples X through XVI described below, which were conducted using just the topical corticosteroid cream and thus are not in accordance with the method of the present invention (note that example VII using the method of using the composition of the present invention can be compared with example IX which does not). That is, the identical experiment was conducted for the same condition but without preceding (or following) the application of the corticosteroid cream with an application of any solution in according with the method of the present invention. The result in each case was that the skin condition improved although not dramatically in anywhere from approximately five to approximately nine minutes and this relief lasted for approximately one to approximately two hours.

Although experiments have only been done on a limited number of topical corticosteroid ointments and lotions, as opposed to creams (for which numerous experiments have been done), in accordance with the method of the present invention, it is believed that the same or similar enhancement effect would be found. In addition, it is believed that the treatment of the present invention would work on any portion of a patient's skin and the above experiments were conducted on the patient's face, legs, arms, chest, scalp, back and other places.

Based upon Applicant's research and experimentation, it is believed that the decongestants naphazoline HCL or phenylephrine HCL are acting with topical corticosteroid cream and the antihistamine pheniramine maleate synergistically. It is not believed that the decongestants are merely increasing the absorbability of the steroid because of two reasons. First, when the effectiveness of hydrocortisone 2.5% alone was compared to the effectiveness of hydrocortisone 0.5% whose application is preceded by the application of either of the decongestants alone (naphazoline HCL alone or phenylephrine HCL alone), the result is that the latter performs better. This indicates that the decongestants are not merely acting as a substitute for increased concentrations and it also indicates that their presence is necessary in the combination used in the method of the present invention. In addition, the fact that each of these two decongestants alone, even with no steroid cream, works somewhat to alleviate dermatological symptoms, albeit not fantastically, further confirms that the decongestants are contributing their own medical benefits rather than merely increasing the absorbability of the steroid cream. It also confirms that the decongestants are necessary in the combination used in the method of the present invention.

Similarly, the fact that the decongestants enhance the effectiveness of the steroid creams albeit mildly even without the antihistantime pheniramine maleate in the treatment of eczema, seborrheic dermatitis, psoriasis and allergic skin reactions further confirms that the decongestants are necessary in the combination used in the method of using the composition of the present invention.

Furthermore, although Applicant has not conducted experiments using only a topical corticosteroid cream that incorporates the antihistamine pheniramine maleate and either naphazoline HCL or phenylephrine HCL (combining the steroid and the decongestant and antihistamine in one cream) in accordance with the principles of the present invention, a person skilled in the art can easily conduct such an experiment. That person would simply formulate such an appropriate composition and then omit the first step of the method and in the second step of the method utilize and apply a composition that already incorporates the combination of antihistamine and decongestant suggested by and used in the first step of the method described herein.

It should also be noted that although Applicant has not performed experiments with the composition of the present invention specifically on psoriasis, Applicant believes that the method and composition of the present invention is likely to be similarly effective on psoriasis since psoriasis is an acute or extreme form of seborrheic dermatitis, for which the present invention has been shown to work. Furthermore, the medications prescribed for psoriasis are the same as those for seborrheic dermatitis.

It is to be understood that while the composition of this invention has been described and illustrated in detail, the above-described embodiments are simply illustrative of the principles of the invention. It is to be understood also that various other modifications and changes may be devised by those skilled in the art which will embody the principles of the invention and fall within the spirit and scope thereof. It is not desired to limit the invention to the exact steps, construction and/or operation shown and described.

## Claims

1. A composition for treating dermatological conditions not known to be caused by a virus or bacteria selected from eczema, seborrheic dermatitis, psoriasis, allergic skin reactions, hives, skin irritations, dermatological conditions caused by poison ivy, dermatological conditions caused by poison oak, comprising:
a cream whose active ingredients comprise
(1) naphazoline HCL or phenylephrine HCL,
(2) pheniramine maleate,
(3) a topical corticosteroid.

2. The composition of claim 1, wherein the topical corticosteroid is hydrocortisone.

3. The composition of claim 1, wherein the topical corticosteroid is selected from halobetasol propionate, desoximetasone, betamethasone diproprionate, beta methasone valerate and triamcinolone acetonide.

## Patentansprüche

1. Zusammensetzung zur Behandlung von dermatologischen Leiden, von denen nicht bekannt ist, dass sie von einem Virus oder von Bakterien verursacht werden, ausgewählt aus Ekzem, seborrhoischer Dermatitis, Psoriasis, allergischen Hautreaktionen, Nesselsucht, Hautirritationen, durch Giftefeu hervorgerufenen dermatologischen Leiden, durch Gifteiche hervorgerufenen dermatologischen Leiden, umfassend:
eine Creme, deren Wirkstoffe umfassen:
1) Naphazolin-HCL oder Phenylephrin-HCL,
2) Pheniraminmaleat,
3) ein topisches Corticosteroid.

2. Zusammensetzung nach Anspruch 1, wobei es sich beim topischen Corticosteroid um Hydrocortison handelt.

3. Zusammensetzung nach Anspruch 1, wobei das topische Corticosteroid aus Halobetasolpropionat, Desoximetason, Betamethasondiproprionat, Betamethasonvalerat und Triamcinolonacetonid ausgewählt ist.

## Revendications

1. Composition pour le traitement d'états dermatologiques non connus pour être provoqués par un virus ou une bactérie, choisis parmi l'eczéma, la parakératose séborrhéique, le psoriasis, les réactions cutanées allergiques, l'urticaire, les irritations cutanées, les états dermatologiques provoqués par le sumac vénéneux, les états dermatologiques provoqués par le sumac à feuilles de chêne, comprenant :
une crème dont les ingrédients actifs comprennent
(1) de la naphtazoline-HCl ou de la phényléphrine-HCl,
(2) du maléate de phéniramine,
(3) un corticostéroïde à usage topique.

2. Composition selon la revendication 1, dans laquelle le corticostéroïde à usage topique est l'hydrocortisone.

3. Composition selon la revendication 1, dans laquelle le corticostéroïde à usage topique est choisi parmi le propionate d'halobétasol, la désoxyméthasone, le dipropionate de bêtaméthasone, le valérate de bêtaméthasone et le triamcinolone acétonide.
